(19) Europäisches Patentamt
European Patent Office
Office européen des brevets

(11) Numéro de publication : **0 500 426 A1**

(12) **DEMANDE DE BREVET EUROPEEN**

(21) Numéro de dépôt : **92400393.2**

(22) Date de dépôt : **13.02.92**

(51) Int. Cl.[5] : **C07D 211/90,** C07D 405/12, A61K 31/44

(30) Priorité : **20.02.91 FR 9102021**

(43) Date de publication de la demande : **26.08.92 Bulletin 92/35**

(84) Etats contractants désignés : **AT BE CH DE DK ES FR GB GR IT LI LU MC NL PT SE**

(71) Demandeur : **Instituto de Investigacion Y Desarrollo Quimico-Biologico S.A.
Avenida de la Industria, 17
E-28100 Alcobendas (Madrid) (ES)**

(72) Inventeur : **Galiano Ramos, Joaquin Alvaro
Tebas No 23
Las Rozas (Madrid) (ES)**
Inventeur : **Del Sol Moreno, Gregorio
La Baneza, 48 P1 E1 6o A
Madrid (ES)**

(74) Mandataire : **Peaucelle, Chantal et al
Cabinet Armengaud Aine 3, avenue Bugeaud
F-75116 Paris (FR)**

(54) **Dérivés de 2,6-diméthyl-4-(4'-hydroxy-3',5'-di-t-butylphényl)-1,4-dihydropyridine, leur procédé de préparation et leur utilisation comme capteurs de radicaux libres.**

(57) L'invention concerne des dérivés de 2,6-diméthyl-4-(4′-hydroxy-3′,5′-di-t-butylphényl)-1,4-dihydropyridine de formule (I) :

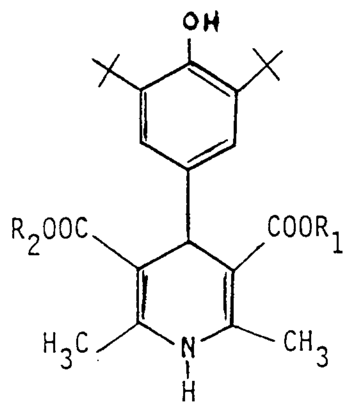

(I)

où :

— $R_1$ est un radical $C_1$-$C_5$ alcoyle linéaire, ramifié ou cyclique, substitué ou non substitué ;

— $R_2$ est un atome d'hydrogène ou un radical $C_1$-$C_5$ alcoyle linéaire ou ramifié, non substitué ou substitué,

leurs isomères et leurs sels pharmaceutiquement acceptables d'addition avec des acides minéraux ou organiques, leur procédé de préparation et leur utilisation comme capteurs de radicaux libres.

EP 0 500 426 A1

L'invention concerne de nouveaux dérivés de 2,6-diméthyl-4-(4′-hydroxy-3′, 5′-di-t-butylphényl)-1,4-dihydropyridine de formule (I) :

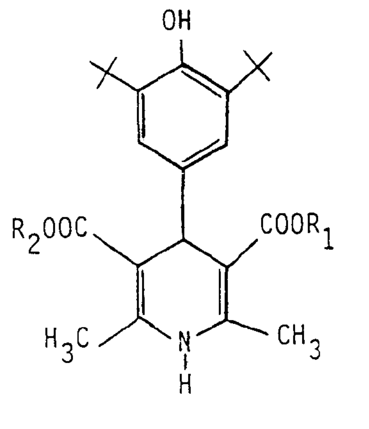

(I)

où :

– $R_1$ est un radical $C_1$-$C_5$-alcoyle linéaire, ramifié ou cyclique, non substitué ou substitué tel que méthyle, éthyle, propyle, isopropyle, cyclopropyle, butyle, isobutyle ou méthoxyéthyle,

– $R_2$ est un atome hydrogène ou un radical $C_1$-$C_5$-alcoyle linéaire ou ramifié, tel que méthyle, éthyle, propyle, isopropyle, butyle, isobutyle, non substitué ou substitué par un ou plusieurs radicaux hydroxyle, phényle, amino, amino substitué ou dioxolanyle, de même que leurs isomères et leurs sels d'addition avec des acides minéraux ou organiques pharmaceutiquement acceptables.

Les composés de l'invention sont chimiquement apparentés aux dérivés de 1,4-dihydropyridine connus pour leur activité antagoniste du calcium. Toutefois, au contraire de ces derniers composés, ils n'ont qu'une faible activité comme bloquants des canaux calciques, mais se sont révélés être de puissants capteurs de radicaux libres.

Lors de l'étude de nouvelles dihydropyridines actives comme bloquants des canaux calciques, l'un des auteurs de la présente invention a observé que le dérivé de 1,4-dihydropyridine de la Fig. 1 :

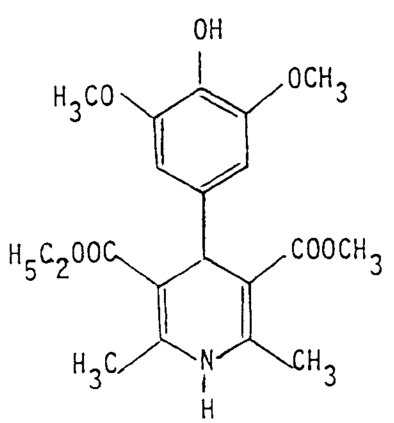

(I)

figure 1

qui porte un radical hydroxyle à la position 4′ du radical phényle est pratiquement exempt d'activité de blocage des canaux calciques (voir brevet espagnol n°531.033), mais manifeste une intéressante activité comme capteur de radicaux libres. Cette propriété s'explique par le fait que ce composé est à même de capter l'électron non apparié des radicaux libres dérivés de l'oxygène en devenant lui-même un radical libre. Néanmoins, du fait que l'électron non apparié peut être délocalisé dans toute la molécule, ce qui donne naissance à plusieurs structures de résonance, comme le montre la Fig. 2, le radical libre correspondant est très stable et ne réagit pas avec d'autres molécules, ce qui arrête la réaction en chaîne.

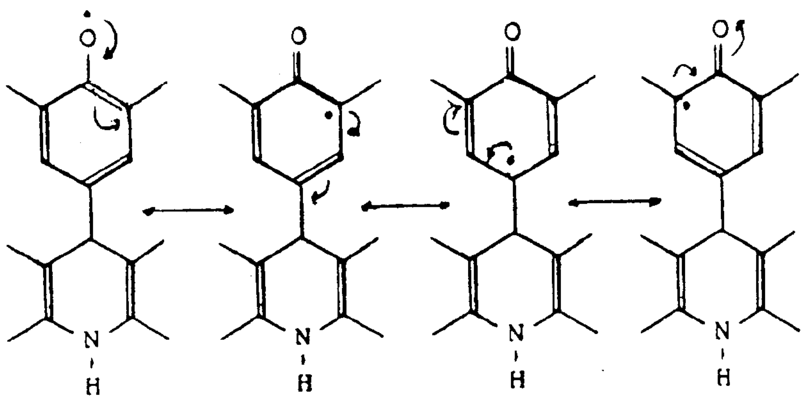

figure 2

La remplacement des radicaux méthoxy en 3′ et 5′ dans le composé de la Fig.1 par des radicaux t-butyle augmente encore l'activité, peut-être parce que leur effet stérique favorise davantage la délocalisation, dans toute la structure, de l'électron non apparié que la réaction avec d'autres molécules de structure de résonance centrée sur l'oxygène.

La présente invention concerne donc aussi l'utilisation des composés de formule (I) comme capteurs de radicaux libres, plus spécialement dans l'application à la prévention des lésions induites par la formation de radicaux libres dérivés de l'oxygène dans certains états pathologiques tels que l'infarctus du myocarde, l'attaque et d'autres états ischémiques, en particulier lorsque une ischémie aiguë est suivie d'une reperfusion.

En fait, les lésions organiques dues à l'ischémie sont la cause fondamentale de mortalité et de morbidité résultant d'une affection vasculaire. Du fait que les manifestations de la lésion sont observées après une certaine durée d'ischémie, il est habituel de qualifier ce processus lésionnel d'ischémique. Néanmoins, bien que cette terminologie puisse convenir pour des organes soumis à de longues durées d'ischémie chronique, la plupart des états cliniques d'ischémie aiguë sont suivis de la reperfusion ou bien de la mort de l'organe et/ou de l'organisme. Par conséquent, les organes qui survivent à une période d'ischémie aiguë auront été soumis tant à une période d'ischémie qu'à une période de reperfusion. Dès lors, il est plus approprié d'appeler le processus complet :"ischémie-reperfusion" et de traiter chaque composante comme une entité distincte. Cette distinction doit être faite en raison de nombreux indices récents montrant que, souvent, une composante essentielle de la lésion ne se manifeste pas pendant la période d'ischémie et, par conséquent, n'est pas due à l'ischémie proprement dite. Une partie importante des lésions n'ont pas lieu pendant l'ischémie, mais plutôt, pendant la reperfusion. Des études sur le mécanisme fondamental de la lésion par reperfusion suggèrent qu'il est étonnamment semblable dans beaucoup d'organes et que la compréhension du mécanisme et sa maîtrise pourraient bien constituer la base de progrès majeurs en thérapeutique.

Les médiateurs les mieux connus de la lésion par reperfusion sont les métabolites toxiques de l'oxygène moléculaire ($O_2$) qui représentent des états intermédiaires de sa réduction électrochimique en eau. Ces intermédiaires, qui sont l'anion superoxyde ($O_2^-$), le peroxyde d'hydrogène ($H_2O_2$) et le radical hydroxyle ($OH^-$) sont très instables et donc très réactifs. Par exemple, le superoxyde et l'hydroxyle sont des radicaux libres ou molécules contenant un électron non apparié dans l'orbitale extérieure. Du fait que cette configuration électronique est très instable, de telles espèces tendent à réagir rapidement avec d'autres molécules en donnant ou acceptant des électrons pour stabiliser leur propre configuration. Ces réactions peuvent mener à dus altérations profondes de la configuration de molécules adjacentes, comme la peroxydation des lipides de la membrane cellulaire conduisant à la rupture de ces cellules. Les radicaux libres peuvent aussi dénaturer les enzymes et l'acide hyaluronique (membranes de base) et produire des altérations sélectives (mutagènes) ou massives (létales) des acides nucléiques. Beaucoup de ces réactions non seulement produisent une perturbation directe dans les molécules adjacentes, mais de plus, en donnant l'électron non apparié supplémentaire à ces molécules, engendrent des espèces radicalaires secondaires qui sont elles-mêmes capables de propager le processus de dégradation. Par conséquent, un radical libre unique peut amorcer une réaction en chaîne conduisant à une lésion tissulaire relativement massive.

Dans les conditions normales, l'oxygène moléculaire est réduit par le système cytochrome dans la mitochondrie et du fait que ce processus n'implique pas d'intermédiaires avec des électrons non appariés dans la couche extérieure, des espèces radicalaires libres ne se forment pas. Néanmoins, même dans les conditions normales, une proportion faible (de l'ordre de 1 à 5%) de l'oxygène réduit en eau à l'intérieur de la cellule subit

la réduction par un trajet univalent avec production de faibles quantités d'intermédiaires radicalaires libres toxiques. Pour lutter contre les dangers réels constitués par ces espèces, les cellules aérobies ont développé des mécanismes de régulation qui réagissent avec les radicaux libres et mettent fin à leur réaction un chaîne. Des exemples de capteurs de radicaux libres sont la superoxyde dismutase, qui est une enzyme hautement spécifique qui catalyse la dismutation du radical superoxyde en peroxyde d'hydrogène, et les catalases et peroxydases qui catalysent la réduction du peroxyde en eau, en court-circuitant la formation intermédiaire du radical hydroxyle. D'autres mécanismes de défense endogènes font intervenir le glutathion réduit et les chélateurs de métaux, comme la transferrine, l'hémoglobine et la myoglobine. D'autres agents, comme l'$\alpha$-tocophérol, sont connus comme étant des capteurs de radicaux libres. Bien que ces agents soient naturellement présents et limitent la production des radicaux libres dans les conditions normales, dans certains cas, leur administration exogène peut être utile pour permettre à la cellule de maîtriser les taux anormaux de production de radicaux libres résultant d'un processus pathologique.

Au cours des quelques dernières années, beaucoup d'attention a été consacrée au développement de nouvelles entités chimiquement une activité pour capter les radicaux libres pour éviter les lésions tissulaires et organiques succédant à des épisodes ischémiques dans le coeur, le cerveau, les reins etc. Des enzymes naturelles telles que la superoxyde dismutase et la catalase ont été produites suivant la technologie de l'ADN recombiné et certains composés chimiques, comme les 21-aminostéroïdes, les dérivés du tocophérol, la ferritine, outre certains flavonoides et antioxydants naturels et synthétiques ont été annoncés comme étant pharmacologiquement utiles dans la prévention des lésions induites par les radicaux libres lors de la reperfusion.

Les composés de la présente invention qui sont sans parenté chimique avec ces capteurs de radicaux libres connus, ont manifesté de façon étonnante une puissante activité dans ce domaine.

La présente invention concerne aussi un procédé de préparation des composés de formule (I), qui consiste à faire réagir le 3,5-di-t-butyl 4-hydroxybenzaldéhyde avec un ester d'acide acétoacétique de formule (II) :

$$H_3C\text{-}CO\text{-}CH_2\text{-}COOR_1 \quad (II)$$

puis à faire réagir l'ester d'acide $\alpha$-acétyl- $\beta$-(3,5-di-t-butyl-4-hydroxy)phénylacrylique résultant de formule (III):

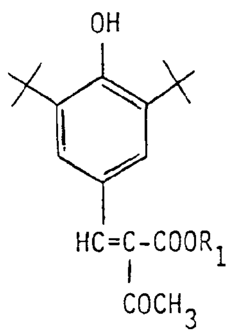

(III)

avec un ester d'acide 3-aminocrotonique de formule (IV):

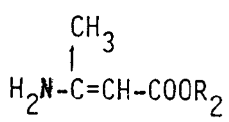

(IV)

Les esters d'acide acétoacétique de formule (II) sont obtenus par réaction du dicétène avec l'alcool approprié.

La présente invention a aussi pour objet les compositions pharmaceutiquement acceptables de composés de formule (I) qui peuvent être administrées par voie orale, rectale, nasale, sublinguale ou par injection. Ces compositions pharmaceutiques sont des mélanges des constituants actifs, facultativement sous la forme d'un sel pharmaceutiquement acceptable, en association avec un excipient pharmaceutiquement acceptable qui peut être un diluant solide, semi-solide ou liquide ou une capsule à ingérer, et une telle composition constitue également un objet de l'invention.

Les composés de formule (I) peuvent aussi être appliqués topiquement pour empêcher ou traiter les lésions dermatologiques produites par une irradiation, des agents chimiques et d'autres situations permettant la formation de radicaux libres.

Habituellement, les substances actives forment entre 0,1% et 99% du poids de la composition, par exemple 0,5% à 10% pour les compositions destinées à l'injection, entre 10% et 80% pour les compositions destinées

à l'usage oral et entre 5% et 90% pour les compositions destinées à l'usage topique.

Pour produire les compositions pharmaceutiques contenant un composé de l'invention sous la forme de doses unitaires à usage oral, le composé actif peut être mélangé avec un excipient pulvérulent solide, par exemple du lactose, du saccharose, du sorbitol, etc., un amidon tel que l'amidon de maïs, l'amylopectine, la gélose, etc., un dérivé cellulosique, de la polyvinylpyrrolidone ou de la gélatine, et peut aussi être additionné de lubrifiants comme le stéarate de magnésium, le carbowax ou d'autres cires de polyéthylèneglycol et pressé en comprimés ou noyaux de pilules.

Dans le cas des pilules, les noyaux peuvent être enrobés, par exemple de solutions concentrées de sucre qui peuvent contenir de la gomme arabique, du talc ou du dioxyde de titane, ou bien d'un agent filmogène en solution dans un solvant organique volatil. Des colorants peuvent être ajoutés à ces revêtements, par exemple pour faire la distinction entre différentes substances actives. Pour la préparation des capsules de gélatine molle, la substance active peut être dissoute dans une huile appropriée, comme l'huile d'olive, l'huile de sésame ou l'huile d'arachide. Les capsules de gélatine dure peuvent contenir des granules de substance active avec des excipients pulvérulents solides tels que le lactose, la saccharose, les amidons, les dérivés cellulosiques, la polyvinylpyrrolidone ou la gélatine, et peuvent aussi contenir du stéarate de magnésium ou de l'acide stéarique à titre de lubrifiants.

Les composés de l'invention peuvent aussi être présentés sous des formes à libération progressive ou à action prolongée, en utilisant des excipients appropriés. Différents procédés sont applicables à la régulation de la disponibilité, par exemple microgranules ou particules enrobés, médicaments insérés dans une matrice ou formes faiblement solubles.

Les préparations liquides pour l'administration par voie orale peuvent avoir la forme d'élixirs, de sirops ou de suspensions, par exemple de solutions contenant environ 0,1% à 10% en poids de substance active, du sucre et un mélange d'alcool, d'eau et de glycérol, du propylèneglycol et éventuellement des aromatisants, de la saccharine et/ou de la carboxyméthylcellulose ou de la pectine comme agents dispersants.

Pour l'administration par voie parentérale, certains des composés de l'invention propres à former des sels avec des acides comme l'acide chlorhydrique, l'acide phosphorique, l'acide tartrique ou d'autres acides organiques ou minéraux peuvent être présentés sous forme de solutions aqueuses du principe actif conforme à l'invention, de préférence à des concentrations de 0,1 à 0,5%, contenant éventuellement un agent stabilisant et/ou tampon. Des doses unitaires de la solution peuvent être avantageusement conditionnées dans des ampoules ou fioles. Pour l'application topique en dermatologie, les composés de l'invention peuvent être présentés en solution alcoolique ou non alcoolique, dans des solutions, émulsions ou sprays, ou être incorporés à des formes cosmétiquement acceptables comme des crèmes, shampooings, gels, crèmes solaires, etc.

La dose à laquelle les constituants actifs sont administrés peut varier dans un certain intervalle et dépend de différents facteurs, par exemple, les besoins individuels du patient. Les doses appropriées s'échelonnent de 10 à 500mg à donner 1 à 3 fois par jour. Les doses appropriées pour l'administration parentérale s'échelonnent de 5 à 250mg. Dans le cas de l'application topique, deux ou trois applications de compositions contenant jusqu'à 10% des composés de l'invention peuvent être nécessaires pour protéger la peau contre les lésions par les radicaux libres.

La préparation des composés conformes à l'invention est illustrée par les exemples suivants.

EXEMPLE 1.-

Préparation du 2,6-diméthyl-4-(4'-hydroxy-3', 5'-di-t-butylphényl)-1,4-dihydropyridine-3,5-dicarboxylate de méthyle et d'éthyle

a) $\alpha$ -Acétyl-$\beta$-(4-hydroxy-3,5-di-t-butylphényl) acrylate d'éthyle.

On introduit 7,03g (30 millimoles) de 3,5-di-t-butyl-4-hydroxybenzaldéhyde et 3,90g (30 millimoles) d'acéto-acétate d'éthyle dans 15 ml de benzène sec dans un ballon à fond rond contenant du benzène sec et raccordé à un séparateur de Dean & Stark. On chauffe le mélange jusqu'à ce que la température atteigne 80°C, puis on y ajoute 0,24 ml de pipéridine et 0,375 ml d'acide acétique glacial.

On chauffe la solution résultante au reflux jusqu'à ce qu'il ne se sépare plus d'eau dans le piège de Dean & Stark (2 heures). Après refroidissememt on dilue le mélange avec 70 ml d'éther, on sépare l'aldéhyde inchangé par filtration et on lave le filtrat avec 2 x 25 ml d'acide chlorhydrique à 5%, avec 2 x 25 ml de bicarbonate de sodium à 5% et enfin avec 25 ml d'eau. On décante la couche organique, on la sèche sur du sulfate de magnésium anhydre et on l'évapore sous vide. On cristallise l'huile résultante dans de l'éther de pétrole. On recristallise l'acrylate dans l'isopropanol.
Rendement : 8 g (76%) d'un solide cristallin blanc

P.F. = 101 - 103°C
IR (KBr) :
3575 $cm^{-1}$ (-OH); 1727 $cm^{-1}$ (C=O); 1670 $cm^{-1}$ (C=O du radical céto);
1649 $cm^{-1}$ (C=C)
RMN ($Cl_3CD$) (δ en ppm) :
7,7-7,1 (M, 3H, 2CH aromatique + 1CH cycle DHP)
5,2 (S, 1H, -OH)
4,4-4 (double Q, 2H, $-CH_2$ du radical éthyle)
2,3 (S, 3H, $-CO-CH_3$)
1,15 (S, 18H, t-butyle)
1,15-0,9 (T, 3H, $CH_3$ du radical éthyle)
RMN ($Cl_3CD$ + THF) (δ en ppm)
7,6 (S, 1H, CH)
7,2 (S, 2H, aromatique)
4-4,4 (Q, 2H, $CH_2$ du radical éthyle)
2,3 (S, 3H, $CO-CH_3$)
1,15 (S, 18H, t-butyle)
1,15-0,9 (T, 3H, $CH_3$ du radical éthyle).

b) 2,6-Diméthyl-4-(4′-hydroxy-3′,5′-di-t-butylphényl)-1,4-dihydropyridine-3,5-dicarboxylate de méthyle et d'éthyle.

On dissout 1 g (2,88 millimoles) d'α-acétyl-β-(4-hydroxy-3,5-di-t-butyl phényl)acrylate d'éthyle et 0,34 g (2,95 millimoles) de 3-aminocrotonate de méthyle dans 25 ml d'isopropanol sec. On laisse la solution reposer pendant 24 heures à 50°C, puis on en chasse le solvant sous vide. On ajoute un supplément de 25 ml d'isopropanol et on laisse la solution reposer à nouveau pendant 24 heures à 50°C. On chasse à nouveau le solvant. On cristallise le produit huileux résiduel dans l'acétate d'éthyle/éther de pétrole (10-90).
Rendement : 0,38 g (30%)
P.F. : 102-104°C
IR (KBr):
3645 $cm^{-1}$, 3602 $cm^{-1}$, 3357 $cm^{-1}$ (bandes -OH et -NH);
1718 $cm^{-1}$ et 1685 $cm^{-1}$ (radicaux C=O); 1654 $cm^{-1}$ (C=C)
RMN ($Cl_3CD$) (δ en ppm)
7 (S, 2H, aromatique)
5,9 (S, 1H, NH)
4,95 (S, 2H, -OH et 4-CH)
4,35-3,9 (Q, 2H, $-CH_2$ du radical éthyle)
3,65 (S, 3H, ester méthylique)
2,35 (S, 6H, 2,6-$CH_3$)
1,2 (S, 18H, du radical t-butyle)
1,2-1,1 (T, 3H, $-CH_3$ du radical éthyle).

EXEMPLE 2.-

Préparation du 2,6-diméthyl-4-(4′-hydroxy-3′,5′-di-t-butylphényl)-1,4-dihydropyridine-3,5-dicarboxylate d'éthyle et d'isopropyle.

On dissout 1,5 g (4,33 millimoles) d'α -acétyl- β -(4-hydroxy-3,5-di-t-butylphényl) acrylate d'éthyle et 0,629 g (4,39 millimoles) de 3-aminocrotonate d'isopropyle dans 30 ml d'isopropanol sec. On laisse la solution reposer 48 heures à 50°C, et après 24 heures au réfrigérateur, on sépare l'acrylate résiduel. On évapore la solution sous vide et on cristallise l'huile résiduelle dans l'éther de pétrole.
Rendement : 0,54 g (27%).
P.F. = 128°-130°C.
IR (KBr) :
3600 $cm^{-1}$, 3300 $cm^{-1}$, (bandes - OH et -NH); 1680 $cm^{-1}$ (C=O);
1645 $cm^{-1}$ (C=C),
RMN ($Cl_3CD$) (δ en ppm)
7,1 (S, 2H, aromatique)

5,7 (S, 1H, NH)
4,9-5 (S, 2H, -OH et 4-CH)
4,5-3,9 (M, 3H, $-CH_2$ des radicaux éthyle et isopropyle)
2,35 (S, 6H, 2,6-$CH_3$)
1,8-0,9 (M, 27H, des radicaux t-butyle et méthyle).

EXEMPLE 3.

Préparation du 2,6-diméthyl-4-(4′-hydroxy-3′,5′-di-t-butylphényl)-1,4-dihydropyridine-3,5-dicarboxylate de diméthyle

a)α - Acétyl-β-(4-hydroxy-3,5-di-t-butylphényl)acrylate de méthyle.

On introduit 7,03 g (30 millimoles) de 4-hydroxy-3,5-di-t-butylbenzaldéhyde et 3,48 g (30 mitlimoles) d'acétoacétate de méthyle dans 15 ml de benzène sec dans un ballon à fond rond raccordé à un séparateur de Dean & Stark contenant du benzène sec. On chauffe le mélange jusqu'à ce que la température atteigne 80°C, puis on y ajoute 0,24 ml de pipéridine et 0,375 ml d'acide acétique glacial. On chauffe la solution résultante au reflux pendant 2 heures jusqu'à ce qu'on n'observe plus de dégagement d'eau. Après refroidissement, on dilue le mélange avec 70 ml d'éther, on sépare par filtration l'aldéhyde inchangé et on lave le filtrat avec 2 x 25 ml d'acide chlorhydrique à 5%, 2 x 25 ml de bicarbonate de sodium à 5% et enfin 25 ml d'eau. On décante la couche organique, on la sèche sur du sulfate de magnésium anhydre et on l'évapore sous vide. On cristallise l'huile résultante dans l'éther de pétrole et on la recristallise dans l'éther diisopropylique.
Rendement : 6,5 g (65%)
P.F. = 99-101°C
IR (KBr) :
3580 $cm^{-1}$ (OH); 2950 $cm^{-1}$ (C aliphatique); 1720 $cm^{-1}$ et
1660$cm^{-1}$ (radicaux C=O); 1640 $cm^{-1}$ (C=C)
RMN ($Cl_3CD$) (δ en ppm)
7,65 et 7,4 (2S, 1H,CH)
7,3 et 7,1 (2S, 2H, aromatique)
5,5 (S, 1H, OH)
3,85 et 3,8 (2S, 3H, $OCH_3$)
2,3 (S, 3H, CO-$CH_3$)
1,3 (S, 18H, $C(CH_3)_3$)
RMN($Cl_3CD$ + THF) (δ en ppm)
7,4 (S, 1H, CH)
7,0 (S, 2H, aromatique)
3,6 (S, 3H, $OCH_3$)
2,3 (S, 1H, CO-$CH_3$)
1,2 (S, 18H, $C(CH_3)_3$)

b) 2,6-Diméthyl-4-(4′-hydroxy-3′,5′-di-t-butylphényl)-1,4-dihydropyridine-3,5-dicarboxylate de diméthyle.

On prépare ce composé par réaction de l'α-acétyl- β -(4-hydroxy-3,5-di-t-butylphényl)acrylate de méthyle et du 3-aminocrotonate de méthyle suivant le procédé décrit dans l'exemple 1b. On cristallise le produit dans l'éther diisopropylique.
Rendement : 43%
P.F. = 191-193°C
IR (KBr) :
3644 $cm^{-1}$ (OH); 3311 et 3241 $cm^{-1}$ (NH); 2951 $cm^{-1}$ (aliphatique);
1699 $cm^{-1}$ (C=O); 1651 $cm^{-1}$ (C=C).
RMN ($Cl_3CD$) (δ en ppm)
7,1 (S, 2H, aromatique)
5,5-6 (S, 1H, NH)
3,7 (S, 6H, d'esters méthyliques)
2, 35 (S, 6H, 2,6-$CH_3$)
1,7-1,3 (S, 18H, $C(CH_3)_3$)

EXEMPLE 4.

Préparation du 2,6-diméthyl-4-(4′-hydroxy-3′,5′-di-t-butylphényl)-1,4-dihydropyridine-3,5-dicarboxylate d'éthyle et de (2,2-diméthyl-1,3-dioxolan-4-yl)méthyle.

a) Acétoacétate de (2,2-diméthyl-1,3-dioxolan-4-yl)méthyle.

On chauffe jusqu'à 75-85°C, 13,06g (99 millimoles) de diisopropylidène glycérol et 36mg d'acétate de sodium anhydre dans un ballon à fond rond muni d'un agitateur magnétique, d'un condenseur à reflux, d'un thermomètre et d'une ampoule d'admission. On ajoute ensuite goutte à goutte 7,67 ml (100 millimoles) de cétène dimère en maintenant la température entre 75 et 85°C. Au terme de l'addition, on agite le mélange pendant une heure à la même température. On isole l'acétoaétate par distillation.
Rendement : 12,8 g (60%)
P.Eb = 142-144°C/15 mm Hg
IR (KBr)
2988 $cm^{-1}$ (aliphatique); 1747 $cm^{-1}$ et 1722 $cm^{-1}$ (C=O)
RMN ($Cl_3CD$) ($\delta$ en ppm)
4,7-3,75 (M, 5H, O-CH et O-$CH_2$)
3,6 (S, 2H, C-$CH_2$-C)
2,3 (S, 3H, $CH_3$-CO)
1,4 (D, 6H, $CH_3$-C-$CH_3$)

b) 3-Aminocrotonate de (2,2-diméthyl-1,3-dioxolan-4-yl)- méthyle.

On dissout 10 g (46 millimoles) d'acétoacétate de(2,2-diméthyl-1,3-dioxolan-4-yl)-méthyle dans 10 ml de méthanol sec dans un ballon équipé d'un système d'admission de gaz. On refroidit la solution dans un bain d'eau glacée et on fait barboter un courant d'ammoniac sec dans le mélange réactionnel jusqu'à saturation (3 heures). On recristallise le solide jaune insoluble dans le n-hexane (solide blanc).
Rendement : 4g (40%)
P.F. = ramollissement à 78°C, fusion à 86°C
IR (KBr) :
3432 $cm^{-1}$ et 3328 $cm^{-1}$ ($NH_2$); 1661 $cm^{-1}$ (C=O); 1616 $cm^{-1}$ (C=C).
RMN ($Cl_3CD$) ($\delta$ en ppm)
4,25 (S, 1H, -C=CH)
4,2-3,3 (M, 5H, O-CH et $OCH_2$)
2 (S, 3H, = C-$CH_3$)
1,3 (D, 6H, $CH_3$-C-$CH_3$)

c) 2,6-Diméthyl-4-(4′-hydroxy-3′,5′-di-t-butylphényl)-1,4-dihydro pyridine-3,5-dicarboxylate d'éthyle et de (2,2-diméthyl-1,3-dioxolan-4-yl)-méthyle.

On dissout 3,46 g (10 millimoles) d'$\alpha$-acétyl-$\beta$-(4-hydroxy-3,5-di-t-butylphényl)acrylate d'éthyle et 2,15g (10 millimoles) de 3-aminocrotonate de(2,2-diméthyl-1,3-dioxolan-4-yl-méthyle dans 50 ml d'isopropanol. On laisse la solution reposer pendant 48 heures à 50°C. On chasse le solvant sous vide et on ajoute un supplément de 50 ml d'isopropanol. Après 24 heures à 50°C, on refroidit le mélange réactionnel au réfrigérateur pendant 24 heures et on filtre l'acrylate résiduel. On chasse le solvant et on cristallise l'huile résiduelle dans l'acétate d'éthyle/éther de pétrole (10-90), puis on la lave avec de l'éther diisopropylique chaud.
Rendement : 2,5 g (46%).
P.F. = 142-145°C
IR (KBr) :
3600 $cm^{-1}$ (OH); 3339 $cm^{-1}$ (NH); 2962 $cm^{-1}$ (aliphatique);
1690$cm^{-1}$ (C=O); 1656 $cm^{-1}$ (C=C)
RMN ($Cl_3CD$) ($\delta$ en ppm)
7,05 (S, 2H, aromatique)
5,8 (S, 1H, NH)
5 et 4,9 (2S, 2H, OH, et 4-CH)
4,65-3,35 (M, 7H, $CH_2$O-et CH-O-)
2,3-2,4 (2S, 6H, 2,6-$CH_3$)

1,3-0,8 (S+T, 21H, $C(CH_3)_3$ et $CH_3$)

EXEMPLE 5.-

Préparation du 2,6-diméthyl-4-(4′-hydroxy-3′,5′-di-t-butyl- phényl)-1,4-dihydropyridine-3,5-dicarboxylate d'éthyle et de 2,3-dihydroxypropyle.

On dissout 0,8 g (1,4 millimole) du composé de l'exemple 4 dans 10 ml d'éthanol à 99% et 2 gouttes d'acide chlorhydrique 11,37N, puis on chauffe le mélange au reflux pendant une heure. On chasse le solvant sous vide et on dissout l'huile résultante dans de l'éther éthylique, puis on filtre le tout. Par évaporation de la solution éthérée, on obtient un solide jaune. Pour la purification, on élue le solide sur une colonne chromatographique de gel de silice, d'abord avec de l'éther diisopropylique pour éliminer les impuretés, puis avec de l'acétone pour éluer la dihydropyridine.
Rendement : 0,32 g (45%)
P.F. = décomposition
IR (KBr):
3630 $cm^{-1}$ et 3337 $cm^{-1}$ (OH et NH); 2959 $cm^{-1}$ (CH aliphatique);
1683 $cm^{-1}$ (C=O); 1622 $cm^{-1}$ (C=C).
RMN ($Cl_3CD$) ($\delta$ en ppm)
7,2 (S, 2H, aromatique)
5,8 (S, 1H, NH)
5 et 4,95 (2S, 2H, OH et 4-CH)
4,6-3 (M, 7H, $CH_2$-O- et CH-O-)
2,3-2,5 (2S, 6H, 2,6-$CH_3$)
2,1 (S, 2H, 2-OH)
1,5-0,9 (S+T, 21H, $C(CH_3)_3$ et $CH_3$)

EXEMPLE 6.-

Préparation du chlorhydrate de 2,6-diméthyl-4-(4′-hydroxy-3′,5′-di-t-butylphényl)-1,4-dihydropyridine-3,5-dicarboxylate d'éthyle et de 2-(N-méthyl-N-benzylamino)éthyle.

a) 2,6-diméthyl-4-(4′-hydroxy-3′,5′-di-t-butylphényl)-1,4-dihydropyridine-3,5-dicarboxylate d'éthyle et de 2-(N-méthyl-N-benzylamino)éthyle, (Base).

On prépare ce composé à partir de 3,46 g (10 millimoles) d'$\alpha$-acétyl-$\beta$-(4-hydroxy-3,5-di-t-butylphényl) acrylate d'éthyle et de 2,48 g (10 millimoles) de 3-aminocrotonate de 2(N-méthyl-N-benzylàmino)éthyle suivant le procédé décrit dans l'exemple 4c, pour obtenir une huile qu'on utilise au stade suivant sans aucune autre purification.
IR (KBr)
3637 $cm^{-1}$ (OH); 3330 $cm^{-1}$ (NH); 2955 $cm^{-1}$ (CH aliphatique);
1693 et 1622 $cm^{-1}$ (C=O des radicaux ester)
RMN ($Cl_3CD$) ($\delta$ en ppm)
7,3 (S, 5H, aromatique)
7,0 (S, 2H, aromatique)
5,65 (S, 1H, NH)
5,0 (S, 2H, OH et 4-CH)
3,8-4,5 (M, 4H, O-$CH_2$)
3,55 (S, 2H, N-$CH_2$-$C_6H_5$)
2,5-3 (T, 2H, $CH_2$-N)
2,3 (S, 6H, 2,6-$CH_3$)
2,25 (S, 3H, $CH_3$-N)
1,35 (S, 21H, $C(CH_3)_3$ et $CH_3$)

b) Chlorhydrate de 2,6-diméthyl-4-(4′-hydroxy-3′,5′-di-t- butylphényl) -1,4-dihydropyridine-3,5-dicarboxylate d'éthyle et de 2(N-méthyl-N-benzylamino)éthyle.

On dissout dans 50 ml de chloroforme le 2,6-diméthyl-4-(4′-hydroxy-3′,5′-di-t-butylphényl)-1,4-dihydropyridine-3,5-dicarboxylate d'éthyle et de 2-(N-méthyl-N-benzylamino)éthyle sous forme de base huileuse. On refroidit la solution au bain d'eau glacée, puis on y ajoute goutte à goutte 0,8 ml d'acide chlorhydrique 11,37N dans du chloroforme et on agite le mélange pendant une heure. On chasse le solvant et on cristallise l'huile résiduelle dans l'éther de pétrole. Après filtration, on met le solide en suspension et on l'agite dans de l'acide chlorhydrique 0,5N, puis on l'extrait dans du chloroforme. On lave la solution chloroformique avec de l'eau, on la sèche sur du sulfate de sodium anhydre et on en chasse le solvant sous vide pour obtenir 2,3 g (37,5% par rapport à l'acrylate) d'un solide jaunâtre qui se révèle avoir une pureté de 98% par HPLC et par potentiométrie.
P.F. = ramollissement à 77°C, fusion à 90°C.
IR (KBr) :
3600-2300 $cm^{-1}$ (plusieurs bandes de OH, de chlorhydrate d'amine, de NH du cycle pyridine); 1694 rt 1683 $cm^{-1}$ (C=O des radicaux ester)
RMN ($Cl_3CD$) (δ en ppm)
7,4 (M, 5H, aromatique)
6,9 (S, 2H, aromatique)
6,1 (S, 1H, NH)
4,9 (S, 1H, OH)
4,8 (S, 1H, CH)
4,5 (S, 2H, $CH_2$-$C_6H_5$)
4,4-3 (M, 6H, O-$CH_2$ et N-$CH_2$)
2,4 (S, 3H, N- $CH_3$)
2,3 (S, 6H, 2,6-$CH_3$)
1-1,6 (S, 21H, $C(CH_3)_3$ et $CH_3$)

EXEMPLE 7.-

Préparation du chlorhydrate de 2,6-diméthyl-4-(4'-hydroxy-3',5'-di-t-butylphényl)-1,4-dihydropyridine-3,5-dicarboxylate d'éthyle et de 2-(méthylamino)éthyle.

On introduit 3,5 g (5,7 millimoles) de chlorhydrate de 2,6-diméthyl-4-(4'-hydroxy-3',5'-di-t-butylphényl)-1,4-dihydropyridine -3,5-dicarboxylate d'éthyle et de 2-(N-méthyl-N-benzylamino)éthyle et 1g de charbon palladié à 10% dans 110 ml de méthanol, dans un ballon de 250 ml équipé pour l'hydrogénation catalytique. On maintient le mélange réactionnel sous atmosphère d'hydrogène à la pression de $7.10^4$ Pa pendant une heure sous agitation. Ensuite, on filtre le mélange sur papier filtre Whatman 40 pour séparer le catalyseur et on évapore le solvant sous vide pour obtenir un solide huileux jaune qu'on triture dans de l'éther isopropylique et qu'on recueille par filtration. En vue de la purification, on chromatographie le produit sur du gel de silice. La première élution avec de l'éther isopropylique élimine les impuretés Ensuite, l'élution au moyen de chloroforme/acétone (5:1) donne 2,5 g (83%) de produit pur.
P.F. = ramollissement à 55°C, fusion à 66°C
IR (KBr):
3600-2300 $cm^{-1}$ (plusieurs bandes de OH, de chlorhydrate d'amine, de NH du cycle pyridine); 1694 et 1679 $cm^{-1}$ (C=O des radicaux ester).
RMN ($Cl_3CD$) (δ en ppm)
6,9 (S, 2H, aromatique)
6,3-6,9 (M, 2H, $^+NH_2$)
6,2 (S, 1H, NH)
4,8-4,6 (S, 1H, OH)
4,8 (S, 1H, 4-CH)
3,6-4,5 (M, 4H, 2 $CH_2O$)
2,8-3,2 (M, 2H, $CH_2$-N)
2,15-2,5 (D, 6H, 2,6-$CH_3$)
2,1 (S, 3H, N-$CH_3$)
0,8-1,5 (M, 21H, $CH_3$, et $C(CH_3)_3$)

Par addition d'eau deutériée à l'échantillon pour RMN, les signaux suivants disparaissent : 6,3-6,9 ppm, 6,2 ppm et 4,8-4,6 ppm. Les signaux qui correspondent aux protons de OH et NH apparaissent avec le signal de $D_2O$ à 2,15 ppm.

EXEMPLE 8.-

Préparation du 2,6-diméthyl-4-(4′-hydroxy-3′,5′-di-t-butylphényl)-1,4-dihydropyridine-3,5-dicarboxylate d'éthyle et de benzyle.

On prépare ce composé par réaction de 3,46 g (10 millimoles) d'α-acétyl-β-(4-hydroxy-3,5-di-t-butylphényl)acrylate d'éthyle et de 1,91 g (10 millimoles) de 3-aminocrotonate de benzyle suivant le procédé décrit dans l'exemple 4c. On dissout le produit huileux brut dans de l'éther isopropylique et on sépare l'acrylate inchangé par filtration. Après élimination du solvant, on élue le produit huileux brut sur une colonne chromatographique de gel de silice avec un mélange d'éther de pétrole et d'éther isopropylique.
Rendement : 3,4 g (65%)
P.F.= ramollissement à 55°C, fusion à 65°C
IR (KBr) :
3638 $cm^{-1}$ (OH); 3337 $cm^{-1}$ (NH); 3089 $cm^{-1}$ (CH aromatique);
2957 $cm^{-1}$ (CH aliphatique); 1683 et 1651 $cm^{-1}$ (C=O des radicaux ester).
RMN ($Cl_3CD$) (δ en ppm)
7, 25 (S, 5H, aromatique)
7,05 (S, 2H, aromatique)
5,8 (S, 1H, NH)
5,15 (S, 2H, $CH_2$ du radical benzyle)
5,05 (S, 1H, OH)
5 (S, 1H, 4-CH)
3,9-4,4 (M, 2H, $CH_2$ de l'aster éthylique)
2,35 (D, 6H, 2,6-$CH_3$)
1-1,6 (M, 21H, $C(CH_3)_3$ et $CH_3$)

EXEMPLE 9.-

Préparation de la 2,6-diméthyl-4-(4′-hydroxy-3′,5′-di-t-butylphényl)-5-carbéthoxy-3-carboxy-1,4-dihydropyridine.

On introduit 2,59 g (5 millimoles) de 2,6-diméthyl-4-(4′-hydroxy-3′,5′-di-t-butylphényl)-1,4-dihydropyridine-3,5-dicarboxylate d'éthyle et de benzyle en solution dans 80 ml de méthanol sec et 0,284 g de charbon palladié à 10% dans un ballon de 250 ml équipé pour l'hydrogénation catalytique, On agite le mélange réactionnel sous atmosphère d'hydrogène à la pression de $21.10^4$ Pa pendant 4 heures. Ensuite, on filtre le mélange sur du papier filtre Whatman 40 pour séparer le catalyseur et on chasse le solvant sous vide pour obtenir un résidu jaunâtre qu'on lave à l'éther isopropylique et ensuite à l'éther éthylique.
Rendement : 1 g (46%) d'un solide blanc
P.F. = 170-171,5°C
IR (KBr) :
3631 - 2500 $cm^{-1}$ (plusieurs bandes de OH, de NH du cycle pyridine et d'acide carboxylique); 1683 $cm^{-1}$ (C=O d'ester); 1652 $cm^{-1}$ (C=O d'acide carboxylique).
RMN ($(CD_3)_2SO$) (δ en ppm)
8,7 (S, 1H, OH)
7 (S, 2H, aromatique)
6,6 (S, 1H, NH)
4,8 (S, 1H, CH)
4,3-3,8 (M, 2H, $CH_3$)
2,3 (S, 6H, 2,6-$CH_3$)
1,6-0,8 (M, 21H, $C(CH_3)_3$ et $CH_3$)

En plus des bandes mentionnées ci-dessus, le spectre RMN comprend les bandes suivantes : 3,1-3,6 ppm et 0,9-1,2 ppm de l'éther éthylique résiduel.

Lorsqu'on ajoute de l'eau deutériée à l'échantillon pour RMN, les signaux de OH et NH disparaissent et sont remplacés par les signaux de $D_2O$ à 4 ppm.

EXEMPLE 10.-

Préparation du 2,6-diméthyl-4-(4'-hydroxy-3',5'-di-t-butylphényl)-1,4-dihydropyridine-3,5-dicarboxylate d'éthyle et d'isobutyle.

On dissout 1,74 g (5 millimoles) d'α-acétyl-β-(4-hydroxy-3,5-dit-butylphényl)acrylate d'éthyle et 0,78 g (5 millimoles) de 3-aminocrotonate d'isobutyle dans 10 ml d'isopropanol. On maintient le mélange pendant 4 jours à 50°C à l'étuve et on chasse ensuite le solvant sous vide. On additionne l'huile résiduelle d'éther isopropylique et on la refroidit au réfrigérateur pendant 24 heures. Ensuite, on sépare l'acrylate inchangé par filtration. On concentre la solution sous vide et on élue la dihydropyridine brute sur une colonne chromatographique de gel de silice avec de l'éther de pétrole pour séparer les impuretés, puis avec de l'éther de pétrole/éther isopropylique pour éluer le produit. On obtient celui-ci sous la forme d'une huile limpide qu'on laisse reposer pendant 48 heures pour la cristallisation. Après agitation avec de l'éther de pétrole, on filtre le solide blanc.
Rendement : 0,75 g (31%)
P.F. = 107,5-108,5°C
IR (KBr) :
3607 $cm^{-1}$ (OH); 3307 $cm^{-7}$ (NH); 2959 $cm^{-1}$ (aliphatique); 1695 et 1684 $cm^{-1}$ (C=O de radicaux ester); 1652 $cm^{-1}$ (C=C).
RMN ($Cl_3CD$) (δ en ppm)
7 (S, 2H, aromatique)
5,8 (S, 1H, NH)
5 (M, 2H, OH et 4-CH)
4,3-3,6 (D, 4H, $CH_2$-O)
2,6-2,3 (D, 6H, 2,6-$CH_3$)
1,7-1,1 (M, 21H, $C(CH_3)_3$ et $CH_3$)
0,7-1,1 (D, 6H, $(CH_3)_2$-C)

EXEMPLE 11.-

Préparation du 2,6-diméthyl-4-(4'-hydroxy-3',5'-di-t-butylphényl)-1,4-dihydropyridine-3,5-dicarboxylate de diisobutyle.

a) α-Acétyl-β-(4-hydroxy-3,5-di-t-butylphényl)acrylate d'isobutyle.

On met 11,7 g (50 millimoles) de 4-hydroxy-3,5-di-t-butylbenzaldéhyde et 8,3 g (52,5 millimoles) d'acétoacétate d'isobutyle en suspension dans 25ml de benzène sec dans un ballon à fond rond raccordé à un séparateur de Dean & Stark contenant du benzène sec. On chauffe le mélange jusqu'à ce que la température atteigne 80°C, puis on y ajoute 1ml de pipéridine et 1,1 ml d'acide acétique glacial. On chauffe la solution résultante au reflux jusqu'à ce qu'on n'observe plus de dégagement d'eau. Après refroidissement, on dilue le mélange avec 50ml d'éther et on le lave avec 2x25ml d'acide chlorhydrique à 5%, 2x25ml de bicarbonate de sodium à 5% et enfin 1x25 ml d'eau. On décante la couche organique, on la sèche sur du sulfate de sodium anhydre et on l'évapore sous vide. On cristallise l'huile résultante dans le n-hexane.
Rendement : 11,5 g (61%)
P.F. = 83-84°C
IR (KBr) :
3584 $cm^{-1}$ (OH); 2959 $cm^{-1}$ et 2873 $cm^{-1}$ (aliphatique); 1728 et 1688 $cm^{-1}$ (C=O); 1660 $cm^{-1}$ (C=C)
RMN ($Cl_3CD$) (δ en ppm)
7,7 et 7,6 (2S, 1H, CH)
7,35 et 7,4 (2S, 2H, aromatique)
5,3-6 (M, 1H, OH)
4,2-4 (2D, 2H,-O-$CH_2$)
2,45 (S, 3H, CO-$CH_3$)
2,3-1,8 (M, 1H, CH du radical isobutyle)
1,5 (S, 18H, $C(CH_3)_3$)
1,1-0,8 (2D, 6H, $CH_3$ du radical isobutyle)
RMN ($Cl_3CD$ + TFA) (δ en ppm)
7,8 (S, 1H, CH)
7,3 (S, 2H, aromatique)

3,8-4,1 (D, 2H, $CH_2$)
2,6 (S, 3H, CO-$CH_3$)
2,3-1,8 (M, 1H, CH du radical isobutyle)
1,5 (S, 18H, $C(CH_3)_3$)
1,1-0,8 (D, 6H, $CH_3$ du radical isobutyle).

b) Préparation du 2,6-diméthyl-4-(4′-hydroxy-3′,5′-di-t-butylphényl)-1,4-dihydropyridine-3,5-dicarboxylate de diisobutyle.

On prépare ce composé par réaction de 7,48 g (20 millimoles) d'α-acétyl-β-(4-hydroxy-3,5-di-t-butylphényl)acrylate d'isobutyle et de 3.14 g (20 millimoles) de 3-aminocrotonate d'isobutyle suivant le procédé décrit dans l'exemple 10 pour obtenir une huile qu'on cristalise dans le n-hexane et l'éther isopropylique.
Rendement : 3,16 g (31%), solide blanc
P.F. = 173-174°C
IR (KBr) :
3590 $cm^{-1}$ (OH); 3368 $cm^{-1}$ (NH); 2965 $cm^{-1}$ (aliphatique);
1679 $cm^{-1}$ (C=O)
RMN ($Cl_3CD$) (δ en ppm)
7,1 (S, 2H, aromatique)
5,9 (S, 1H, NH)
5 (S, 1H, OH)
4,95 (S, 1H, 4-CH)
4-3,8 (D, 4H, -$OCH_2$)
2,3 (S, 6H, 2,6-$CH_3$)
2,2-1,7 (M, 2H, CH du radical isobutyle)
1,4 (S, 18H, $C(CH_3)_3$).

Les exemples suivants illustrent comment les composés de l'invention peuvent être incorporés à des compositions pharmaceutiques.

EXEMPLE 12.-

Comprimés

Chaque comprimé contient :

| | |
|---|---|
| Substance active | 50 à 500,0 mg |
| Amidon de maïs | 25,0 mg |
| Lactose | 90,0 mg |
| Gélatine | 1,5 mg |
| Talc | 12,5 mg |
| Stéarate de magnésium | 1,5 mg |

EXEMPLE 13.-

Capsules molles.

Chaque capsule contient :

| | |
|---|---|
| Substance active | 50 à 500,0 mg |
| Glycérine | 150,0 mg |
| Polyoxyéthylèneglycol 400 | 50,0 mg |
| Eau distillée | 150,0 mg |
| Saccharine | 2,0 mg |

EXEMPLE 14.-

Solution.

| | |
|---|---|
| Substance active | 1 à 5,0 g |
| Alcool à 96° | 20,0 ml |
| Glycérol | 10,0 ml |
| Propylèneglycol | 2,0 ml |
| Parfum | 0,5 ml |
| Eau distillée q.s. | 100,0 ml |

EXEMPLE 15.-

Crème.

| | |
|---|---|
| Substance active | 1 à 5,0 g |
| Alcool cétylique | 2,0 g |
| Monostéarate de glycérol | 8,0 g |
| Spermacéti | 5,0 g |
| Tween 60 | 3,0 g |
| Palmitate d'isopropyle | 2,0 g |
| Méthylparabène | 0,2 g |
| Propylparabène | 0,02 g |
| Eau distillée q.s. | 100, 0 g |

Pharmacologie.

Les composés de la présente invention ont fait l'objet d'un essai de contrôle de leur aptitude à capter les radicaux libres engendrés par la membrane hépatique du rat stimulée par du Fe++/acide ascorbique (système

connu pour induire une formation de radicaux libres). Les composés les plus actifs ont fait ensuite l'objet d'essais de contrôle de leur aptitude à inhiber la formation des radicaux libres par les neutrophiles humains stimulés par la N-formyl-L-méthionyl-L-leucyl-L-phénylalanine et à protéger le tissu hépatique, entretenu en culture, contre les lésions par ischémie/reperfusion.

1.- Activité de captage des radicaux libres dans la membrane hépatique du rat.

On a préparé des homogènats de foie de rat (1/10 p/v) dans du Ringer-Tris (50mM)-saccharose (0,32 mM) de pH 7,4, après sacrifice de rats Wistar au moyen de $CO_2$. On a découpé les foies en petits morceaux (1 à 2 mm) et on les a homogénéisés à 0°C dans un homogénéisateur Polytron. On a centrifugé les homogénats à 1000 x g pendant 10 minutes à 4°C et rejeté le culot. On a centrifugé le surnageant à 10 000 x g pendant 20 minutes et recueilli le culot, puis on l'a remis en suspension, dans le rapport 1/10, dans du Ringer-Tris (50 mM). On a utilisé des échantillons de la préparation immédiatement et on en a conservé à -20°C. On a induit la peroxydation des lipides dans des tubes contenant des membranes hépatiques de rat en suspension dans 1,7 ml de Ringer-Tris (20mM) (1/4 v/v) et 100 µl de différentes concentrations des composés de l'invention ou d'$\alpha$-tocophérol, de dipyridamol ou d'hydroxytoluène butylé, par addition de 100 µl de sulfate ferreux (100 M) et d'acide ascorbique (100 M). Après 45 minutes à 37°C, on a ajouté 1 ml d'une solution contenant 0,5% d'acide thiobarbiturique (ATB) dans de l'acide trichloroacétique (ATC) à 20% dans chaque tube et après 15 minutes d'incubation à 95°C, on a déterminé par spectrophotométrie les substances réagissant avec l' ATB. On a réalisé pour toutes les expériences des témoins appropriés exempts du médicament.

2.- Dosage des radicaux libres dans les polymorphonucléaires.

On a isolé des neutrophiles humains chez des donneurs volontaires bien portants par centrifugation sur gradient dans du Ficol Hypaque (Pharmacia). On a mélangé 100 µl de suspension de cellules (nombre final 500 000 cellules) avec 700 µl de solution physiologique tamponnée au phosphate et 50 µl des composés expérimentaux en solution dans du DMSO. On a utilisé comme témoin positif 25 µl d'une solution à 1 mg par ml de superoxyde dismutase dans du DMSO (à savoir 25 µg de SOD). On a préincubé les tubes à 37°C pendant 10 minutes sous agitation lente.

On a stimulé les neutrophiles par addition de 100 µl de cytochrome C type VI (1 mM) (Sigma) dans de la solution physiologique tamponnée au phosphate, de 50 µl de N-formyl-L-méthionyl-L-leucyl-L-phénylalanine (FMPLA) (10 µM) (Sigma F 3506) et par incubation pendant 5 minutes à 37°C. Après refroidissement à 0°C pour arrêter la réaction, on a centrifugé les tubes à 8000 xg et on a déterminé par spectrophotométrie à 550 nm le cytochrome C réduit, en utilisant des tubes contenant du SOD comme témoins.

3.- Ischémie/reperfusion dans une culture d'organe à court terme.

Pour ces études, on a prélevé des échantillons de tissu dans des groupes de 10 rats Wistar mâles anesthésiés au pentobarbital sodique (60 mg par kg, i.p.). On a isolé les foies et on les a divisés en cinq échantillons d'environ 0,4 g qu'on a pesés et transférés dans des fioles d'Erlenmeyer de 50 ml bouchées contenant de la solution de Tris-Ringer saturée d'argon (anoxique).

Ischémie

On a purgé à l'argon les fioles d'Erlenmeyer de 50 ml contenant les échantillons du tissu et 3,9 ml de tampon de Tris-Ringer traité à l'argon, on les a rebouchées et on les a conservées au bain-marie à 37°C sans agitation pendant 50 minutes pour provoquer l'ischémie. La subdivision du tissu et l'agitation ont été évitées à ce stade pour réduire au minimum la diffusion des métabolites depuis le tissu jusque dans le milieu, qui n'aurait pu se faire au cours d'une ischémie in vivo.

Division du tissu et addition du médicament.

Après 50 minutes d'ischémie, on a divisé les tissus avec des ciseaux d'ophtalmologie en fragments d'environ 1 mm de côté. Du fait que l'argon est plus dense que l'air, son déplacement hors de la fiole et son remplacement par l'oxygène sont restés minimaux pendant le découpage et l'addition ultérieure du médicament. Ensuite, on a ajouté 0,1 ml de tampon de Tris-Ringer contenant l'un des composés à évaluer ou de l'hydroxytoluène butylé (HTB), pour arriver à la concentration finale souhaitée; on a sélectionné l'hydroxytoluène butylé du fait que c'est un antioxydant classique qui gêne la propagation en chaîne des radicaux. On a procédé à ces

additions avec rapidité pour éviter d'introduire de l'oxygène. On a rebouché les fioles et on les a secouées modérément pour mélanger le tissu avec le médicament, puis on les a remises au bain-marie pendant 10 minutes pour la mise a l'équilibre avant la réoxygénation.

Réoxygénation.

Après 60 minutes d'anoxie ischémique, on a réoxygéné les fioles par barbotage vif d'oxygène gazeux à 100% dans les échantillons pendant 1 minute et par purge de la phase gazeuse avec de l'oxygène (de l'argon remplaçant l'oxygène à ce stade dans les expériences témoins d'ischémie sans réoxygénation). Ensuite, on a bouché toutes les fioles et on les a remises au bain-marie sous agitation pour 60 minutes de réoxygénation à 37°C. Ce procédé est décrit comme une culture de lamelles "en flottement libre" (Dickson et Suzangar 1976 Human Tumors in Short Term Culture. Ed., PP. Dendy, Londres : Academic Press) du fait que les fragments de tissu se trouvent en suspension sans support dans le milieu.

La surface du milieu est d'environ 12 $cm^2$ et sa profondeur est d'environ 3 mm seulement pour amener au minimum la mise à l'équilibre du milieu avec la phase gazeuse supérieure.

Réaction à l'acide thiobarbiturique pour le dosage du malonaldéhyde.

On a dosé le malonaldéhyde par le procédé spectrophotométrique de Buege et Aust (1979) ("Microsomal lipid Peroxidation", Methods in Enzymology, 52, 302-310). On a homogénéisé les contenus des fioles, comprenant le tissu et le tampon, dans un homogénéisateur en Téflon/verre. On a combiné l'homogénat total avec 4,0 ml d' ATC-ATB dans un tube à essai et on l'a mélangé soigneusement. On a couvert chaque tube d'une bille de verre et on l'a chauffé pendant 15 minutes au bain-marie à 90-95°C. Après refroidissement, on a séparé le précipité floculant par 5 minutes de centrifugation à 1500xg. On a déterminé l'absorbance du surnageant par spectrophotométrie à 530 nm par comparaison avec un témoin contenant 4 ml du réactif ATC/ATB, 3,9 ml de tampon et 0,1 ml du médicament correspondant en solution tampon. On a établi les courbes d'étalonnage avec du malonaldéhyde bis-diméthylacétal (MDA). Par analyse de régression, on a calculé les $CI_{50}$ c'est-à-dire les concentrations du médicament inhibant de 50% la production du malonaldéhyde.

Résultats.

1. Activité de captage des radicaux libres dans les membranes hépatiques de rat.

On a observé les $CI_{50}$ ci-après :

| Composés | $CI_{50}$ (µM) |
|---|---|
| Exemple 1 | 0,78 |
| Exemple 2 | 0,29 |
| Exemple 3 | 1,25 |
| Exemple 4 | 1,25 |
| Exemple 5 | 50,00 |
| Exemple 6 | 20,00 |
| Exemple 7 | 2,50 |
| Exemple 8 | 1,00 |
| Exemple 9 | 2,50 |
| Exemple 10 | 0,37 |
| Exemple 11 | 1,00 |
| Dipyridamol | 3,50 |
| α-Tocophérol | 15,00 |
| HTB | 1,50 |

A partir de ces données, on a sélectionné les composés des exemples 1,2 et 10 pour des essais supplémentaires.

2. Effets sur la stimulation des neutrophiles.

La SOD à la concentration utilisée pour l'expérience inhibe totalement la production de radicaux libres dans les neutrophiles humains stimulés par la FMLPA. L'activité des composés sélectionnés conformes à l'invention est :

| Composés | $CI_{50}$ (µM) |
|---|---|
| Exemple 1 | 10,86 |
| Exemple 2 | 10,19 |
| Exemple 10 | 10,48 |
| HTB | 20,0 |

3. Effets sur la production des radicaux libres dans l'ischémie reperfusion.

L'inhibition de la production du MDA, par des composés selon l'invention, dépend de la dose.

| Composés | $CI_{50}$ (µM) |
|---|---|
| Exemple 1 | 0,17 |
| Exemple 2 | 0,2 |
| Exemple 10 | 0,2 |
| HTB | 1,75 |

## Revendications

**1.-** Dérivés de 2,6-diméthyl-4-(4'-hydroxy-3',5'-di-t-butylphényl)-1,4-dihydropyridine de formule (I) :

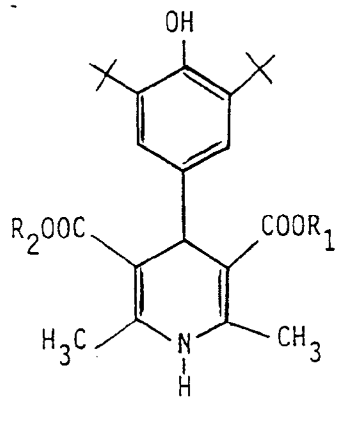

(I)

où :

– $R_1$ est un radical $C_1$-$C_5$-alcoyle linéaire, ramifié ou cyclique, non substitué ou substitué tel que méthyle, éthyle, propyle, isopropyle, cyclopropyle, butyle, isobutyle ou méthoxyéthyle;

– $R_2$ est un atome d'hydrogène ou un radical $C_1$-$C_5$- alcoyle linéaire ou ramifié, tel que méthyle, éthyle, propyle, isopropyle, butyle, isobutyle, non substitué ou substitué par un ou plusieurs radicaux hydroxyle,

phényle, amino, amino substitué ou dioxolanyle, leurs isomères et leurs sels d'addition avec des acides minéraux ou organiques pharmaceutiquement acceptables.

**2.-** Suivant la revendication 1, le 2,6-diméthyl-4-(4'-hydroxy-3',5'di-t-butylphényl)-1,4-dihydropyridine- 3,5-dicarboxylate de méthyle et d'éthyle.

**3.-** Suivant la revendication 1, le 2,6-diméthyl-4-(4'hydroxy-3',5'di-t-butylphényl)-1,4-dihydropyridine-3,5-dicarboxylate d'éthyle et d'isopropyle.

**4.-** Suivant la revendication 1, le 2,6-diméthyl-4-(4'-hydroxy-3',5'di-t-butylphényl)-1,4-dihydropyridine-3,5-dicarboxylate de diméthyle

**5.-** Suivant la revendication 1, le 2,6-diméthyl-4-(4'-hydroxy-3',5'-di-t-butylphényl)-1,4-dihydropyridine-3,5-dicarboxylate d'éthyle et de (2,2-diméthyl-1,3-dioxolan-4-yl)-méthyle.

**6.-** Suivant la revendication 1, le 2,6-diméthyl-4-(4'-hydroxy-3',5'-dit-butylphényl)-1,4-dihydropyridinu-3,5-dicarboxylate d'éthyle et de 2,3-dihydroxypropyle.

**7.-** Suivant la revendication 1, le chlorhydrate de 2,6-diméthyl-4-(4'hydroxy-3',5'-di-t-butylphényl)-1,4-dihydropyridine-3,5-dicarboxylate d'éthyle et de 2-(N-méthyl-N-benzylamino)éthyle.

**8.-** Suivant la revendication 1, le chlorhydrate de 2,6-diméthyl-4-(4'-hydroxy-3',5'-di-t-butylphényl)-1,4-dihydropyridine-3,5-dicarboxylate d'éthyle et de 2-(méthylamino)éthyle.

**9.-** Suivant la revendication 1, le 2,6-diméthyl-4-(4'-hydroxy-3',5'-di-t-butylphényl)-1,4-dihydropyridine-3,5-dicarboxylate d'éthyle et de benzyle.

**10.-** Suivant la revendication 1, la 2,6-diméthyl-4-(4'-hydroxy-3',5'-di-t-butylphényl)-5-carbéthoxy-3-carboxy-1,4-dihydropyridine.

**11.-** Suivant la revendication 1, le 2,6-diméthyl-4-(4'-hydroxy-3'-5'-di-t-butylphényl)-1,4-dihydropyridine-3,5-dicarboxylate d'éthyle et d'isobutyle.

**12.-** Suivant la revendication 1, le 2,6-diméthyl-4-(4'-hydroxy-3',5'-di-t-butylphényl)-1,4-dihydropyridine-3,5-dicarboxylate de diisobutyle.

**13.-** Procédé de préparation des composés de formule I suivant la revendication 1, qui comprend la réaction du 3,5-di-t-butyl-4-hydroxybenzaldéhyde avec un ester d'acide acétoacétique de formule(II):

$$H_3C\text{-}CO\text{-}CH_2\text{-}COOR_1 \quad (II)$$

où $R^1$ est tel que défini dans la revendication 1,
puis la réaction de l'ester d'acide α-acétyl-β-(3,5-di-t-butyl--4-hydroxy)phénylacrylique résultant de formule(III):

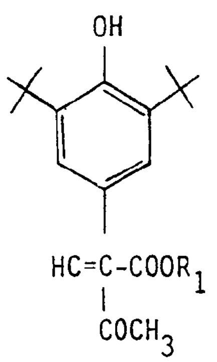

(III)

avec un ester d'acide 3-aminocrotonique de formule(IV):

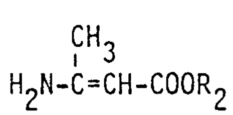

(IV)

où $R_2$ est tel que défini dans la revendication 1.

**14.-** Composé suivant les revendications 1 à 12, utilisé comme capteur de radicaux libres.

**15.-** Composé suivant les revendications 1 à 12, utilisé comme médicament pour le traitement des affections post-ischémiques ou des altérations en relation avec la peroxydation des lipides ou des lésions induites par les radicaux libres.

**16.-** Composition pharmaceutique ou cosmétique comprenant un composé suivant les revendications 1 à 12 comme constituant actif conjointement avec un excipient pharmaceutiquement ou cosmétiquement acceptable.

Office européen des brevets

# RAPPORT DE RECHERCHE EUROPEENNE

Numero de la demande

EP 92 40 0393

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | DE-A-2 335 466 (BAYER AG)<br>* revendications 1,15-17; exemple 19 * | 1,13,16 | C07D211/90<br>C07D405/12<br>A61K31/44 |
| A | DE-A-1 963 185 (FARBENFABRIKEN BAYER AG)<br>* revendications 1-7; exemple 1 * | 1,16 | |
| A | CHEMICAL ABSTRACTS, vol. 103, no. 19, 11 Novembre 1985, Columbus, Ohio, US; abstract no. 160398J, page 700 ;colonne 1 ;<br>* abrégé *<br>& JP-A-60 056 956 (S. OZAKI ET AL) 2 Avril 1985 | 1,16 | |
| A | US-A-3 923 818 (F. BOSSERT ET AL)<br>* colonne 5, ligne 21 - ligne 41 *<br>* colonne 14, ligne 62 - ligne 63 *<br>* colonne 18, ligne 40 - ligne 41 * | 1 | |
| A | FR-A-2 322 196 (INSTITUT ORGANICHESKOGO SINTEZA AKADEMII NAUK LATVIISKOI SSR)<br>* revendications 1-3 * | 1,14 | **DOMAINES TECHNIQUES RECHERCHES (Int. Cl.5 )** |
| A | WO-A-8 301 775 (RIKER LABORATORIES INC)<br>* revendications 1-12 * | 1,14-16 | C07D<br>A61K |

Le présent rapport a été établi pour toutes les revendications

| Lieu de la recherche | Date d'achèvement de la recherche | Examinateur |
|---|---|---|
| BERLIN | 22 AVRIL 1992 | van Amsterdam |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)